(19) Europäisches Patentamt
European Patent Office
Office européen des brevets

(11) Publication number: **0 186 055 B1**

(12) **EUROPEAN PATENT SPECIFICATION**

(45) Date of publication of patent specification: **25.07.90**

(51) Int. Cl.$^5$: **A 61 K 31/415,** A 61 K 47/00

(21) Application number: **85115830.3**

(22) Date of filing: **12.12.85**

# (54) Antimycotic gel preparations.

(30) Priority: **25.12.84 JP 271890/84**

(43) Date of publication of application:
**02.07.86 Bulletin 86/27**

(45) Publication of the grant of the patent:
**25.07.90 Bulletin 90/30**

(84) Designated Contracting States:
**AT BE CH DE FR GB IT LI NL SE**

(56) References cited:
**EP-A-0 007 595**
**EP-A-0 095 813**
**EP-A-0 104 037**
**DE-A-3 244 027**

**CHEMICAL ABSTRACTS, vol. 103, no. 12, 23rd September 1985, page 325, abstract no. 92854c, Columbus, Ohio, US; & JP-A-60 61 518 (HISAMITSU PHARMACEUTICAL CO., INC.) 09-04-1985**

(73) Proprietor: **BAYER AG**
**D-5090 Leverkusen 1 Bayerwerk (DE)**

(72) Inventor: **Uehara, Minehiko**
**12-1, Nishishibukawa 1-Chome**
**Kusatsu-shi Shiga-ken (JP)**
Inventor: **Ohara, Yoshishige**
**Ko 320, Minamitsuchiyama Tsuchiyama-Cho**
**Koka-Gun Shiga-ken (JP)**
Inventor: **Hattori, Toshiyuki**
**77, Tera Gamou-Cho**
**Gamou-Gun Shiga-ken (JP)**
Inventor: **Nishioka, Takaaki**
**1072, Oharanaka Koda-Cho**
**Koka-Gun Shiga-ken (JP)**
Inventor: **Hata, Hiroko**
**1072, Oharanaka Koda-Cho**
**Koka-Gun Shiga-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 186 055 B1

**Description**

This invention relates to antimycotic gel preparations for external use containing clotrimazole or bifonazole, preferably clotrimazole.

Clotrimazole has the chemical name of 1-[(2-chlorophenyl)diphenylmethyl]imidazole, molecular formula $C_{22}H_{17}ClN_2$, molecular weight 344.84, the structure

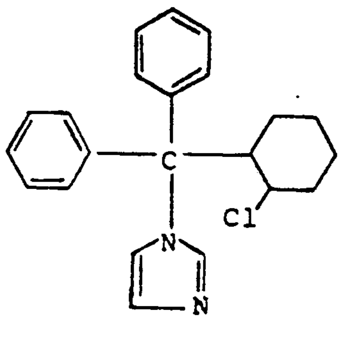

and has a strong antimycotic action. It has excellent therapeutic effects on *Tinea pompholyciformis, Tinea interdigitalis, Tinea crusis* and *Trichophytia meculovesiculosa.*

Bifonazole has the chemical name 1-[α-(4-biphenyl)-benzyl]-imidazol, the structure

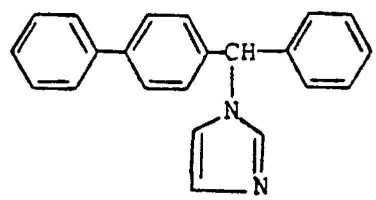

the molecular formula $C_{22}H_{18}N_2$, the molecular weight 310,4 and has a strong antimycotic action.

The present invention provides a bifonazole or clotrimazole gel preparation which permits excellent penentration and migration (absorption) of the active ingredient through the skin, and has a good use feeling and excellent storage stability.

Thus, according to this invention, there is provided an antimycotic gel preparation characterized by containing 0.5—3 wt% of clotrimazole or bifonazole, 0.1—3 wt.% of carboxy vinyl polymer, 0.1—5 wt.% of an organic amine and 5—90 wt.% of 1,3-butylene glycol, ≤ 30 wt.% of ethylalcohol, ≤ 40 wt.% of purified water, all percent by weight figures based on the weight of the preparation, and moreover characterized by a viscosity of 1000 to 5000 at 20°C in Pas and a pH of 7 to 9.5.

1,3-Butylene glycol dissolves clotrimazole and bifonazole fairly well, has a high boiling point and therefore hardly vaporizes from the coated surface, and produces an effect of greatly promoting the penetration and migration of clotrimazole and bifonazole through the skin. In the gel preparation of this invention, 1,3-butylene glycol can be used in a proportion of generally 5 to 90% by weight, preferably 30 to 80% by weight, more preferably 40 to 70% by weight, based on the weight of the preparation.

1,3-Butylene glycol is comparatively oleophilic, and undergoes phase separation when a large quantity of water is added to it. Accordingly, to improve its miscibility with purified water which is optionally used in the gel preparation of this invention, the gel preparation may contain ethyl alcohol as required, which, however, is generally used in an amount of not more than 30% by weight based on the weight of the preparation. Depending upon the amount of purified water used, it is convenient to use ethyl alcohol in a proportion of preferably 5 to 30% by weight, more preferably 10 to 29% by weight.

The gel preparation of this invention uses a carboxy vinyl polymer as a gelling agent. The carboxy vinyl polymers are hydrophilic (or water-soluble) polymers containing a carboxyl group in the molecule and having a number average molecular weight of usually about 1,000,000 to about 3,000,000, and include hydrophilic polymers having acrylic acid as a main structural unit (for the properties and other details of the carboxy vinyl polymers, reference may be made to "Japanese Standards of Cosmetic Ingredients" edited by Japan Association of Official Publications and Committee of Investigations on the Standards of Cosmetic Ingredients in Central Pharmaceutical Affairs Council, Ministry of Health and Welfare, second edition, Explanation I, pages 272—280, published by Yakuji Nippo, Ltd. on August 1, 1984). Specific examples of such a carboxy vinyl polymer are those sold under the trade names Hiviswako 103, 104 and 105 by Wako Pure Chemical Industry Co., Ltd. and those sold under the trade names Carbopol 934, 940 and 941 by B. F. Goodrich Chemical Co. (Cleaveland, Ohio, U.S.A.).

These carboxy vinyl polymers generally have a free carboxyl group. An aqueous solution of the carboxy vinyl polymer shows acidity, and when neutralized with an organic amine, it becomes a viscous gel.

The content of the carboxy vinyl polymer in the gel preparation of this invention is not strict, and can be varied widely according to the contents of the other ingredients. The suitable amount of the polymer is generally 0.1 to 3% by weight, preferably 0.3 to 2% by weight, more preferably 0.4 to 1% by weight, based on the weight of the gel preparation. From the viewpoint of the viscosity of the final gel preparation, it is desirable to adjust the content of the carboxy vinyl polymer so that the viscosity of the gel preparation at 20°C becomes generally 400 to 10,000 Pas preferably 1000 to 5000 Pas.

The organic amine to be used for neutralization of the carboxy vinyl polymer is suitably water-soluble. Specific examples include mono-lower alkanolamines such as monomethanolamine, monoethanolamine, monopropanolamine and monoisopropanolamine; di-lower alkanolamines such as dimethanolamine, diethanolamine, dipropanolamine, diisopropanolamine, dibutanolamine, diisobutanolamine and di-sec-butanolamine; tri-(lower alkanol)amines such as trimethanolamine, triethanolamine, tripropanolamine, triisopropanolamine, tributanolamine, triisobutanolamine and tri-sec-butanolamine; mono-lower alkylamines such as methylamine, ethylamine, propylamine and isopropylamine; di-lower alkylamines such as dimethylamine, diethylamine, dipropylamine and diisopropylamine; and tri-lower alkylamines such as trimethylamine, triethylamine, tripropylamine and triisopropylamine. Of these, the mono- or di-lower alkanolamines are preferred, and mono- or di-iso-propanolamine is most preferred. These amines may be used singly or as a combination of two or more.

The organic amine can be used in an amount required to neutralize the carboxy vinyl polymer at least partly, or in an amount slightly in excess of it. Specifically, it is advantageously used in an amount such that the pH of the final gel preparation becomes 5 to 10, preferably 7 to 9.5. The amount of the organic amine, which depends upon its type, the content of the carboxy vinyl polymer, etc. is generally 0.1 to 5% by weight, preferably 0.2 to 4% by weight, more preferably 0.3 to 3% by weight, based on the weight of the gel preparation.

As required and desirably, the gel preparation of this invention may contain purified water. The term "purified water", as used herein, denotes water obtained by distilling ordinary water or passing it through an ion-exchange resin. Purified water serves as a solvent for the carboxy vinyl polymer and is useful for increasing the affinity of the gel preparation for the skin. Accordingly, purified water is conveniently used in an amount of generally not more than 40% by weight, preferably 3 to 35% by weight, more preferably 5 to 30% by weight, based on the weight of the gel preparation.

The gel preparation of this invention is generally a transparent gel, and its typical compositions and properties are as shown in the following table.

Table 1

| Component | General range (wt.%) | Preferred range (wt.%) | Most preferred range (wt.%) |
|---|---|---|---|
| Clotrimazole | 0.5-3 | 1-2.5 | 1.0-2.0 |
| or | | | |
| Bifonazole | 0.5-3 | 1-2.5 | 1.0-2.0 |
| Carboxy vinyl polymer | 0.1-3 | 0.3-2 | 0.4-1 |
| Organic amine | 0.1-5 | 0.2-4 | 0.3-3 |
| 1,3-Butylene glycol | 5-90 | 30-80 | 40-70 |
| Ethyl alcohol | ≦30 | 5-30 | 10-29 |
| Purified water | ≦40 | 3-35 | 5-30 |
| Total | 100 | 100 | 100 |
| pH | 5—10 | Preferably 7—9.5 | |
| Viscosity (centipoises | 4,000—100,000, preferably 10,000—50,000 | | |
| Pas at 20°C) | 400—10,000 | 1,000—5,000 | |

The gel preparations of this invention can be produced by mixing the aforesaid ingredients with each other until a uniform mixture forms. It can be produced by dissolving clotrimazole or bifonazole in 1,3-butylene glycol, adding the carboxy vinyl polymer to the 1,3-butylene glycol solution, and adding the organic amine to the mixture with stirring.

Preferably, clotrimazole and bifonazole are first dissolved in 1,3-butylene glycol. In the dissolving step, the dissolving can preferably be promoted by heating the materials to about a temperature range of 20°C to 90°C, preferably to 50—85°C, especially preferred to about 70 to 80°C. Then, as required and preferably, ethyl alcohol is added to the 1,3-butylene glycol solution. Furthermore, the carboxy vinyl polymer is added in the form of an aqueous solution or a dispersion in 1,3-butylene glycol and purified water, preferably as the aqueous solution. Thereafter, the organic amine is preferably added in the form of an aqueous solution or an ethyl alcohol solution to the mixture, and they are fully mixed with stirring. The addition of the organic amine led to the neutralization of the carboxy vinyl polymer, and the consistency of the mixture increases. As a result, a generally transparent gel-like preparation having the individual ingredients uniformly dispersed therein is obtained. When the carboxy vinyl polymer is used in the form of an aqueous solution, its suitable concentration is generally 0.5 to 10% by weight, preferably 1 to 5% by weight.

Also preferably the organic amine is added as required and desirably to the 1,3-butylene glycol solution of clotrimazole or bifonazole so prepared, and thereafter, the carboxy vinyl polymer is added in the form of an aqueous solution or a dispersion in 1,3-butylene glycol or a mixture of 1,3-butylene glycol and purified water (preferably as the aqueous solution). They are fully mixed, e.g. with stirring to form the gel preparation of this invention.

The method for the production of an antimycotic gel preparation therefore is also a subject of the present invention. Preferred are the embodiments specifically described above.

The gel preparations provided by this invention permit very good penetration and migration (absorption) of clotrimazole or bifonazole through the skin and exhibit a high level of antimycotic activity. They have a refreshing use feeling, little side-effects such as skin irritation and excellent storage stability. Hence, they are very useful for practical purposes. Such effects of the gel preparations are demonstrated by the following test results obtained by using the gel preparation produced in Example 7 below.

(1) Stability test

The clotrimazole gel preparation of this invention (Example 7) was filled in an aluminum tube (contents 1 g), and stored in a constant temperature/humidity chamber kept at a temperature of 40°C and a relative humidity of 75%. It was regularly taken out, and the amounts of clotrimazole in the gel preparation and the decomposition product of clotrimazole (o-chlorophenylcarbinol) in it were determined by high-performance liquid chromatography. The results are shown in Table 1.

Table 1

| Lot | | 1 | | 2 | | 3 | |
|---|---|---|---|---|---|---|---|
| Test item | | Amount (%) | | Amount (%) | | Amount (%) | |
| | | Clotri-mazole (*1) | Decomp-osition product (*2) | Clotri-mazole | Decomp-osition product | Clotri-mazole | Decomp-osition product |
| Storage period | 4 weeks | 99.2 | 0.1 | 99.4 | 0.2 | 98.8 | 0.2 |
| | 8 weeks | 99.2 | 0.3 | 99.3 | 0.2 | 99.6 | 0.2 |
| | 12 weeks | 99.6 | 0.4 | 100.0 | 0.4 | 100.5 | 0.3 |
| | 20 weeks | 98.5 | 0.7 | 99.3 | 0.6 | 99.0 | 0.5 |
| | 28 weeks | 98.7 | 0.9 | 99.3 | 0.8 | 98.5 | 0.7 |

(*1): Content of clotrimazole

(*2): Content of o-chlorophenylcarbinol (the decomposition product of clotrimazole)

It is seen from Table 1 that when the gel preparation of this invention was stored for 28 weeks at 40°C, the amount of o-chlorophenylcarbinol, the decomposition product of clotrimazole, formed was less than 1%, and it had excellent chemical stability.

(2) Primary skin irritation test

The primary skin irritation rate in rabbits was measured in accordance with the method of Draize et al. (see Draize, J. H., et al. 1944, Methods for the study of irritation and toxicity of substances applied topically to the skin and mucous membranes, J. Pharmacol. Exp. Ther. 83; 377—390). The primary skin irration rate is a value calculated by dividing by 4 the sum of evaluation points of intact (I) skin and abraded (A) skin assigned after the lapse of 24 hours and 72 hours respectively after application of the drug. The results are shown in Table 2 below.

Usually, primary skin irritation rates of at least 2.0 are regarded as indicating the presence of irritation (see Yasuhiko Shirasu et al., Environmental Toxicology: Method and Safety Evaluation, pages 444—449, Soft Science Co., Ltd., 1975).

Table 2

| Rabbit No. | Skin | Erythema | | Edema | | Incrustation | | Total | | Primary skin irritation rate |
|---|---|---|---|---|---|---|---|---|---|---|
| | | After 24 hours | After 72 hours | After 24 hours | After 72 hours | After 24 hours | After 72 hours | After 24 hours | After 72 hours | |
| 1 | Intact (I)<br>Abraded (A) | 2<br>2 | 0<br>0 | 2<br>2 | 0<br>0 | 0<br>0 | 1<br>1 | 8 | 2 | 2.50 |
| 2 | I<br>A | 1<br>1 | 1<br>1 | 0<br>2 | 0<br>1 | 0<br>0 | 0<br>1 | 4 | 4 | 2.00 |
| 3 | I<br>A | 1<br>1 | 0<br>0 | 0<br>1 | 0<br>0 | 0<br>0 | 0<br>0 | 3 | 0 | 0.75 |
| 4 | I<br>A | 1<br>1 | 0<br>0 | 0<br>1 | 0<br>0 | 0<br>0 | 0<br>0 | 3 | 0 | 0.75 |
| 5 | I<br>A | 1<br>1 | 0<br>0 | 0<br>2 | 0<br>0 | 0<br>0 | 0<br>1 | 4 | 1 | 1.25 |
| 6 | I<br>A | 1<br>1 | 0<br>0 | 0<br>2 | 0<br>0 | 0<br>0 | 0<br>1 | 4 | 1 | 1.25 |
| Average value ± standard deviation | | | | | | | | | | 1.42±0.70 |

The results of Table 2 demonstrate that the gel preparation of this invention showed a primary skin irritation rate of 1.42±0.70 (n=6), and thus had little skin irritation.

The following examples further illustrate the present invention. In these examples, ethyl alcohol used was 95% by volume ethyl alcohol, and water used was one purified on an ion-exchange resin (purified water). The viscosity was measured at 20°C by means of a VT-type viscometer (a product of Haake Company, U.S.A.). The carboxy vinyl polymer used was Carbopol 940 produced by B. F. Goodrich Chemical Co.

Example 1

Clotrimazole (1 g) was dissolved in 10 g of 1,3-butylene glycol heated at 65 to 75°C, and 30 g of ethyl alcohol was added to the solution. The mixture was stirred to form a uniform solution. Then, 20 g of a 2.5% aqueous solution of carboxy vinyl polymer was added, and the mixture was well stirred. Then, 9 g of a 10% aqueous solution of diisopropanolamine was added, and further 1,3-butylene glycol was added to adjust the total amount to 100 g. After thorough stirring, a transparent gel preparation was obtained which had a viscosity of 3,200 Pas (32,000 centipoises) and a pH of 9.3.

Example 2

Clotrimazole (1 g) was dissolved in 20 g of 1,3-butylene glycol heated at 65 to 75°C, and 20 g of ethyl alcohol was added to it. The mixture was stirred to form a uniform solution. Then, 12.5 g of a 4% aqueous solution of carboxy vinyl polymer was added, and the mixture was well stirred. To the mixture was added 13 g of a 10% aqueous solution of triisopropanolamine, and 1,3-butylene glycol was further added to adjust the total amount to 100 g. After thorough stirring, a transparent gel preparation was obtained which had a viscosity of 3,000 Pas (30,000 centipoises) and a pH of 8.3.

Example 3

Clotrimazole (1 g) was dissolved in 20 g of 1,3-butylene glycol heated at 65 to 75°C, and 10 g of ethyl alcohol was added. The mixture was stirred, and 1 g of triethanolamine was further added to form a uniform solution. Then, 30 g of a 2.5% aqueous solution of carboxy vinyl polymer was added, and the mixture was well stirred. Furthermore, 1,3-butylene glycol was added to adjust the total amount to 100 g. After thorough stirring, a transparent gel preparation was obtained which had a viscosity of 3,800 Pas (38,000 centipoises) and a pH of 7.4.

Example 4

Clotrimazole (2 g) was dissolved in 40 g of 1,3-butylene glycol heated at 65 to 75°C, and 0.5 g of monoethanolamine was further added to form a uniform solution. To the solution was added 30 g of a 2.5% aqueous solution of carboxy vinyl polymer, and the mixture was well stirred. Furthermore, 1,3-butylene glycol was added to adjust the total amount to 100 g. After thorough stirring, a transparent gel preparation was obtained which had a viscosity of 2,800 Pas (28,000 centipoises) and a pH of 6.5.

Example 5

Clotrimazole (1 g) was dissolved in 30 g of 1,3-butylene glycol heated at 65 to 75°C to form a uniform solution. To the solution was added 40 g of a 2% suspension of carboxy vinyl polymer in 1,3-butylene glycol, and the mixture was well stirred. Eight grams of a 10% aqueous solution of diisopropanolamine was added, and the mixture was well stirred. Furthermore, 1,3-butylene glycol was added to adjust the total amount to 100 g. After thorough stirring, a transparent gel preparation was obtained which had a viscosity of 2,500 Pas (25,000 centipoises) and a pH of 7.5.

Example 6

Clotrimazole (1 g) was dissolved in 10 g of 1,3-butylene glycol heated at 65 to 75°C. After cooling, 10 g of ethyl alcohol was added, and the mixture was stirred to form a uniform solution. Then, 60.5 g of a dispersion of 0.5 g of carboxy vinyl polymer in 40 g of 1,3-butylene glycol and 20 g of purified water was added, and the mixture was well stirred. Then, 18 g of a 5% ethyl alcohol solution of diisopropanolamine was added, and 1,3-butylene glycol was further added to adjust the total amount to 100 g. After thorough stirring, a transparent gel preparation was obtained which had a viscosity of 3,100 Pas (31,000 centipoises) and a pH of 9.0

Example 7

Clotrimazole (1 g) was dissolved in 10 g of 1,3-butylene glycol heated at 65 to 75°C. After cooling, 30 g of ethyl alcohol was added, and the mixture was stirred. Furthermore, 0.9 g of diisopropanolamine was added, and the mixture was stirred to form a uniform solution. Thereafter, 55.5 g of a dispersion of 0.5 g of carboxy vinyl polymer in 35 g of 1,3-butylene glycol and 20 g of purified water was added, and the mixture was stirred. Furthermore, 1,3-butylene glycol was added to adjust the total amount to 100 g. After thorough stirring a transparent gel preparation was obtained which had a viscosity of 3,000 Pas (30,000 centipoises) and a pH of 9.0.

Example 8

Carboxy vinyl polymer (0.8 g) was dissolved in 40 g of 1,3-butylene glycol and 20 g of purified water to form a uniform solution.

Then, 1 g of clotrimazole was dissolved in 10 g of 1,3-butylene glycol heated at 65 to 75°C. After cooling, 25 g of ethyl alcohol was added, and the mixture was stirred. Furthermore, 1.4 g of diisopropanolamine was dissolved, and the resulting solution was added to the carboxy vinyl polymer solution prepared as above. The mixture was stirred, and 1,3-butylene glycol was added to adjust the total amount to 100 g. After thorough stirring, a transparent gel preparation was obtained which had a viscosity of 4,300 Pas (43,000 centipoises) and a pH of 9.0.

Example 9

Carboxy vinyl polymer (0.8 g) was dissolved in 35 g of 1,3-butylene glycol, 15 g of ethyl alcohol and 20 g of purified water to form a uniform solution.

Then, clotrimazole (1 g) was dissolved in 10 g of 1,3-butylene glycol heated at 65 to 75°C. After cooling, 15 g of ethyl alcohol was added, and the mixture was stirred. Furthermore, 1.4 g of diisopropanolamine was dissolved. The resulting solution was added to the carboxy vinyl polymer solution prepared as above, and the mixture was stirred. Furthermore, 1,3-butylene glycol was added to adjust the total amount to 100 g. After thorough stirring, a transparent gel preparation was obtained which had a viscosity of 4,100 Pas (41,000 centipoises) and a pH of 9.0.

Example 10

Carboxy vinyl polymer (0.5 g) was dissolved in 35 g of 1,3-butylene glycol and 20 g of purified water to form a uniform solution.

Then, 1 g of clotrimazole was dissolved in 10 g of 1,3-butylene glycol heated at 65 to 75°C. After cooling, 30 g of ethyl alcohol was added, and the mixture was stirred. Furthermore, 0.9 g of diisopropanolamine was dissolved. The resulting solution was added to the carboxy vinyl polymer solution prepared as above, and the mixture was stirred. Furthermore, 1,3-Butylene glycol was added to adjust the total amount to 100 g. After thorough stirring, a transparent gel preparation was obtained which had a viscosity of 3,050 Pas (30,500 centipoises) and a pH of 9.0.

Example 11

Bifonazole (1 g) was dissolved in 10 g of 1,3-butylene glycol heated at about 70°C and 30 g of ethyl alcohol was added to the solution. The mixture was stirred to form a uniform solution. Then, 20 g of a 2.5% aqueous solution of carboxy vinyl polymer was added, and the mixture was well stirred. Then, 9 g of a 10% aqueous solution of diisopropanolamine was added, and further 1,3-butylene glycol was added to adjust the total amount to 100 g. After thorough stirring a transparent gel preparation was obtained.

Example 12

Bifonazole (1 g) was dissolved in 20 g of 1,3-butylene glycol heated at about 70°C and 20 g of ethyl alcohol was added to it. The mixture was stirred to form a uniform solution. Then, 12.5 g of a 4% aqueous solution of carboxy vinyl polymer was added, and the mixture was well stirred. To the mixture was added 13 g of a 10% aqueous solution of triisopropanolamine, and 1,3-butylene glycol was further added to adjust the total amount to 100 g. After thorough stirring, a transparent gel preparation was obtained.

Example 13

Bifonazole (1 g) was dissolved in 20 g of 1,3-butylene glycol heated at about 70°C and 10 g of ethyl alcohol was added. The mixture was stirred, and 1 g of triethanolamine was further added to form a uniform solution. Then, 30 g of a 2.5% aqueous solution of carboxy vinyl polymer was added, and the mixture was well stirred. Furthermore, 1,3-butylene glycol was added to adjust the total amount to 100 g. After thorough stirring, a transparent gel preparation was obtained.

Example 14

Bifonazole (2 g) was dissolved in 40 g of 1,3-butylene glycol heated at about 70°C and 0.5 g of monoethanolamine was further added to form a uniform solution. To the solution was added 30 g of a 2.5% aqueous solution of carboxy vinyl polymer, and the mixture was well stirred. Furthermore, 1,3-butylene glycol was added to adjust the total amount to 100 g. After thorough stirring, a transparent gel preparation was obtained.

Example 15

Bifonazole (1 g) was dissolved in 30 g of 1,3-butylene glycol heated at about 70°C to form a uniform solution. To the solution was added 40 g of a 2% suspension of carboxy vinyl polymer in 1,3-butylene glycol, and the mixture was well stirred. Eight grams of a 10% aqueous solution of diisopropanolamine was added, and the mixture was well stirred. Furthermore, 1,3-butylene glycol was added to adjust the total amount to 100 g. After thorough stirring, a transparent gel preparation was obtained.

Example 16

Bifonazole (1 g) was dissolved in 10 g of 1,3-butylene glycol heated at about 70°C. After cooling, 10 g of ethyl alcohol was added, and the mixture was stirred to form a uniform solution. Then, 60.5 g of a dispersion of 0.5 g of carboxy vinyl polymer in 40 g of 1,3-butylene glycol and 20 g of purified water was added, and the mixture was well stirred. Then, 18 g of a 5% ethyl alcohol solution of diisopropanolamine was added, and 1,3-butylene glycol was further added to adjust the total amount to 100 g. After thorough stirring, a transparent gel preparation was obtained.

Example 17

Bifonazole (1 g) was dissolved in 10 g of 1,3-butylene glycol heated at about 70°C. After cooling, 30 g of ethyl alcohol was added, and the mixture stirred. Furthermore, 0.9 g of diisopropanolamine was added, and the mixture was stirred to form a uniform solution. Thereafter, 55.5 g of a dispersion of 0.5 g of carboxy vinyl polymer in 35 g of 1,3-butylene glycol and 20 g of purified water was added, and the mixture was stirred. Furthermore, 1,3-butylene glycol was added to adjust the total amount to 100 g. After thorough stirring, a transparent gel preparation was obtained.

Example 18

Carboxy vinyl polymer (0.8 g) was dissolved in 40 g of 1,3-butylene glycol and 20 g of purified water to form a uniform solution.

Then, 1 g of bifonazole was dissolved in 10 g of 1,3-butylene glycol heated at about 70°C. After cooling, 25 g of ethyl alcohol was added, and the mixture was stirred. Furthermore, 1.4 g of diisoproanolamine was dissolved, and the resulting solution was added to the carboxy vinyl polymer solution prepared as above. The mixture was stirred, and 1,3-butylene glycol was added to adjust the total amount to 100 g. After thorough stirring, a transprent gel preparation was obtained.

Example 19

Carboxy vinyl polymer (0.8 g) was dissolved in 35 g of 1,3-butylene glycol, 15 g of ethyl alcohol and 20 g of purified water to form a uniform solution.

Then, bifonazole (1 g) was dissolved in 10 g of 1,3-butylene glycol heated at about 70°C. After cooling, 15 g of ethyl alcohol was added, and the mixture was stirred. Furthermore, 1.4 g of diisopropanolamine was dissolved. The resulting solution was added to the carboxy vinyl polymer solution prepared as above, and the mixture was stirred. Furthermore, 1,3-butylene glycol was added to adjust the total amount to 100 g. After thorough stirring, a transparent gel preparation was obtained.

Example 20

Carboxy vinyl polymer (0.5 g) was dissolved in 35 g of 1,3-butylene glycol and 20 g of purified water to form a uniform solution.

Then, 1 g of bifonazole was dissolved in 10 g of 1,3-butylene glycol heated at about 70°C. After cooling, 30 g of ethyl alcohol was added, and the mixture was stirred. Furthermore, 0.9 g of diisopropanolamine was dissolved. The resulting solution was added to the carboxy vinyl polymer solution prepared as above, and the mixture was stirred. Furthermore, 1,3-butylene glycol was added to adjust the total amount to 100 g. After thorough stirring, a transparent gel preparation was obtained.

**Claims**

1. An antimycotic gel preparation characterized by containing 0.5—3 wt.% of clotrimazole or bifonazole, 0.1—3 wt.% of a carboxy vinyl polymer, 0.1—5 wt.% of an organic amine and 5—90 wt.% of 1,3-butylene glycol, $\leq$ 30 wt.% of ethylalcohol, $\leq$ 40 wt.% of purified water, all percent by weight figures based on the weight figures based on the weight of the preparation, and moreover characterized by a viscosity of 1000 to 5000 at 20°C in Pas and a pH of 7 to 9.5.

2. The gel preparation of any one of Claim 1 wherein the organic amine is a mono- or di-lower alkanolamine, preferably mono- or di-isopropanolamine.

3. The gel preparation of any one of Claims 1 to 2 which is transparent and has a pH of 7 to 9.5 and a viscosity at 20°C of 1000 to 5000 Pas, and consists of 1.0 to 2.0% by weight of clotrimazole or 1.0 to 2.0% by weight of bifonazole, 0.4 to 1% by weight of diisopropanolamine, 40 to 70% by weight of 1,3-butylene glycol, 10 to 29% by weight of ethyl alcohol and 5 to 30% by weight of purified water.

4. Method for the production of an antimycotic gel preparation characterized in that 0.5—3 wt.% of clotrimazole or bifonazole, 0.1—3 wt.% of a carboxy vinyl polymer, 0.1—5 wt.% of an organic amine and 5-90 wt.% of 1,3-butylene glycol, all percent by weight figures based on the weight of the preparation are thoroughly mixed and the pH is adjusted to 7 to 9.5.

**Patentansprüche**

1. Antimykotisches Gel-Präparat, dadurch gekennzeichnet, daß es 0,5 bis 3 Gew.% Clotrimazol oder Bifonazol, 0,1 bis 3 Gew.-% eines Carboxyvinyl-Polymers, 0,1 bis 5 Gew.-% eines organischen Amins und 5

bis 90 Gew.-% 1,3-Butylenglycol, ≤30 Gew.% Ethylalkohol, ≤40 Gew.% gereinigtes Wasser enthält, wobei sämtliche zahlenmäßigen Gew.-%-Angaben auf das Gewicht des Präparats bezogen sind, und weiterhin gekennzeichnet durch eine Viskosität von 1 000 bis 5 000 Pa s bei 20°C und einen pH-Wert von 7 bis 9,5.

2. Gel-Präparat nach Anspruch 1, worin das organische Amin ein Mono- oder Di-niederalkanolamin, vorzugsweise Mono- oder Di-isopropanolamin, ist.

3. Gel-Präparat nach irgendinem der Ansprüche 1 bis 2, das transparent ist, einen pH-Wert von 7 bis 9,5 und eine Viskosität von 1 000 bis 5 000 Pa s bei 20°C hat und aus 1,0 bis 2,0 Gew.-% Clotrimazol oder 1,0 bis 2,0 Gew.-% Bifonazol, 0,4 bis 1 Gew.% Diisopropanolamin, 40 bis 70 Gew.-% 1,3-Butylenglycol, 10 bis 29 Gew.-% Ethylalkohol und 5 bis 30 Gew.-% gereinigtem Wasser besteht.

4. Verfahren zur Herstellung eines antimykotischen Gel-Präparat, dadurch gekennzeichnet, daß 0,5 bis 3 Gew.-% Clotrimazol oder Bifonazol, 0,1 bis 3 Gew.% eines Carboxyvinyl-Polymers, 0,1 bis 5 Gew.-% eines organischen Amins und 5 bis 90 Gew.% 1,3-Butylenglycol, wobei sämtliche zahlenmäßigen Gew.-%-Angaben auf das Gewicht des Präparats bezogen sind, gründlich miteinander vermischt werden und der pH-Wert auf 7 bis 9,5 eingestellt wird.

**Revendications**

1. Préparation de gel antimycotique caractérisée en ce qu'elle contient 0,5 à 3% en poids de clotrimazole ou de bifonazole, 0,1 à 3% en poids d'un polymère carboxyvinylique, 0,1 à 5% en poids d'une amine organique et 5 à 90% en poids de 1,3-butylèneglycol, 30% en poids d'alcool éthylique, 40% en poids d'eau purifiée, tous les pourcentages en poids étant rapportés au poids de la préparation, et caractérisée également par une viscosité de 1000 à 5000 Pas à 20°C et par un pH de 7 à 9,5.

2. Préparation de gel selon la revendication 1, dans laquelle l'amine organique est une mono- ou dialcanolamine inférieure et, de préférence, la mono- ou la diisopropanolamine.

3. Préparations de gel selon l'une des revendications 1 ou 2, qui est transparente et a un pH de 7 à 9,5, avec une viscosité à 20°C de 1000 à 5000 Pa.s et qui est constituée de 1 à 2% en poids de clotrimazole ou de 1 à 2% en poids de bifonazole, de 0,4 à 1% en poids de diisopropanolamine, de 40 à 70% en poids de 1,3-butylèneglycol, de 10 à 29% en poids d'alcool éthylique et de 5 à 30% en poids d'eau purifiée.

4. Procédé pour la production d'une préparation de gel antimycotique, caractérisé en ce que l'on mélange complètement 0,5 à 3% en poids de clotrimazole ou de bifonazole, 0,1 à 3% en poids d'un polymére carboxyvinylique, 0,1 à 5% en poids d'une amine organique et 5 à 90% en poids de 1,3-butylèneglycol, tous les pourcentages en poids étant rapportés au poids de la préparation, et que le pH est réglé pour atteindre 7 à 9,5.